(19) Europäisches Patentamt / European Patent Office / Office européen des brevets

(11) **EP 3 457 914 B1**

(12) **FASCICULE DE BREVET EUROPEEN**

(45) Date de publication et mention de la délivrance du brevet:
**02.07.2025 Bulletin 2025/27**

(21) Numéro de dépôt: **17726852.1**

(22) Date de dépôt: **12.05.2017**

(51) Classification Internationale des Brevets (IPC):
**A61B 5/00** *(2006.01)* **A61B 5/1455** *(2006.01)*

(52) Classification Coopérative des Brevets (CPC):
**A61B 5/0075; A61B 5/14552; A61B 5/6886**

(86) Numéro de dépôt international:
**PCT/EP2017/061508**

(87) Numéro de publication internationale:
**WO 2017/198575 (23.11.2017 Gazette 2017/47)**

(54) **DISPOSITIF MÉDICAL SANS FIL D'ACQUISITION DE VIDÉOS CUTANÉES BIMODALITÉ AVEC CONTRÔLE LUMINEUX**

**DRAHTLOSE MEDIZINISCHE VORRICHTUNG ZUR AUFNAHME BIMODALER HAUTVIDEOS MIT LICHTSTEUERUNG**

**WIRELESS MEDICAL DEVICE FOR ACQUIRING BIMODAL SKIN VIDEOS WITH LIGHT CONTROL**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.05.2016 FR 1654409**

(43) Date de publication de la demande:
**27.03.2019 Bulletin 2019/13**

(73) Titulaire: **Université de Lorraine
54000 Nancy (FR)**

(72) Inventeurs:
• **AMOUROUX, Marine
54425 Pulnoy (FR)**
• **BLONDEL, Walter
54425 Pulnoy (FR)**
• **HILL, Kevin
54130 Saint Max (FR)**
• **HAUDRECHY, Alexandre
54880 Vasberg (FR)**

(74) Mandataire: **IPAZ
Bâtiment Platon
Parc Les Algorithmes
91190 Saint-Aubin (FR)**

(56) Documents cités:
US-A1- 2011 117 025     US-A1- 2014 240 468
US-A1- 2015 124 067     US-A1- 2016 022 181
US-B1- 6 775 565

**Description**

La présente invention concerne un dispositif médical et un procédé d'acquisition de vidéos cutanées.

Le besoin de disposer de l'image des plaies chroniques prises en charge dans le cadre de la télémédecine est satisfait depuis quelques années par l'utilisation de dispositifs « grand public » de type tablettes tactiles, téléphones portables, appareils photos numériques et caméras plafonnier par les infirmières à domicile, les médecins coordonnateur en EHPAD (Etablissement d'Hébergement pour Personnes Âgées Dépendantes) et/ou par des médecins qui requièrent à distance un avis ponctuel de consultant dans le cadre du parcours de soins des plaies chroniques. Une enquête a permis de mettre en évidence la limite de tels appareils car de nombreuses photographies envoyées aux experts ne sont que partiellement exploitables : images floues, à faible indice de rendu colorimétrique ou avec un éclairage insuffisant (certains patients doivent fournir leur lampe de poche personnelle afin d'envoyer des images exploitables aux experts). En effet, l'éclairage environnant qui est le seul éclairage exploité (sauf en cas d'utilisation du flash qui modifie l'indice de rendu colorimétrique et accentue la brillance) n'est pas constant ni en température de couleur corrélée (lumière des néons du plafond, lampe de chevet à incandescence ou lumière solaire si le patient est installé près d'une fenêtre durant une journée ensoleillée) ni en intensité (lumière solaire variant selon la couverture nuageuse, la saison, l'heure de la journée et les équipements au domicile des patients étant variables en nombre et en orientation). L'absence de polarisation impacte également négativement la qualité des images générées car la brillance (réflexion spéculaire) empêche souvent l'expertise à distance des lésions concernées.

A l'heure actuelle, les recommandations internationales relatives à la prise en charge des plaies chroniques en environnement clinique prévoient dans certains cas la mesure de la PtcO$_2$ : Pression transcutanée en dioxygène. Cependant, le paramètre physiologique du niveau d'oxygénation cutanée n'est quasiment jamais mesuré en routine clinique car :

- les dispositifs permettant sa mesure sont chers : au moins 5 000 C, et
- la mesure est chronophage : au moins 30 minutes par patient, pour une information limitée en termes de surface cutanée explorée, la plupart du temps, les dispositifs étant équipés de 3 électrodes de 1 cm$^2$ de surface chacune. Soit la mesure de la PtcO$_2$ sur une surface de 3 cm$^2$ en 30 minutes environ.

On connait le document Kong, L. et al. "Non-contact detection of oxygen saturation based on visible light imaging device using ambient light". Opt. Express 21, 17464-17471 (2013), décrivant un appareil de mesure du niveau d'oxygénation utilisant de la lumière blanche.

On connait également le document Nitzan, M. et al. "Calibration-Free Pulse Oximetry Based on Two Wavelengths in the Infrared - A Preliminary Study." Sensors 14, 7420-7434 (2014), mettant en œuvre l'utilisation de deux longueurs d'onde dans l'infrarouge.

Le document US2004174525 décrit un dispositif de visualisation utilisant une polarisation parallèle et croisée.

Le document US 2013/0071103 décrit un appareil capable de réaliser des séquences vidéos en utilisant plusieurs polariseurs et des diodes électroluminescentes émettant à des longueurs d'ondes différentes.

On connaît le document US 2014/240468 décrivant un système d'imagerie encombrant, fixé au sol qui n'est nullement un dispositif portatif. Ce système permet de réaliser une image obtenue à partir d'un éclairage de plusieurs longueurs d'ondes différentes, en particulier, le rouge, le vert et le bleu. Les ondes proche infra-rouge sont également utilisées.

Le document US 2016/022181 décrit un appareil d'imagerie permettant d'obtenir des images provenant d'un capteur proche infra-rouge et des images provenant d'un capteur dans le visible. Pour ce faire, l'appareil comprend des diodes 12 proche infra-rouge et des diodes 14 dans le visible. Ce document décrit également deux lasers de positionnement permettant de positionner l'appareil par rapport à la surface imagée.

Les documents US 2015/124067 et US 2015/124067 concernent un dispositif portatif comportant au moins un illuminateur pour projeter un faisceau de lumière et une caméra vidéo pour capturer des images d'une région d'intérêt.

Le document US 2011/117025 décrit un dispositif d'imagerie et de surveillance à base de fluorescence. Ce dispositif comprend une source de lumière pour éclairer la cible, la lumière émise comprenant au moins une longueur d'onde ou une bande de longueur d'onde provoquant la fluorescence d'au moins un biomarqueur associé à la cible; et un détecteur de lumière pour détecter la fluorescence.

La présente invention a pour objectif un appareil portatif capable de réaliser rapidement des images de deux types :

- Apportant une information morphologique de bonne qualité c'est-à-dire avec un indice de rendu colorimétrique élevé, une bonne résolution et une bonne netteté et

- Apportant une information fonctionnelle relative au niveau d'oxygénation cutanée.

L'invention a encore pour but un appareil dont le coût de revient est compatible avec un prix de vente lui-même compatible avec un prix à l'achat défini lors d'une enquête comme permettant la vente auprès des futurs clients-cibles (infirmiers à domicile, établissement d'hébergement pour personnes âgées dépendantes (EHPAD), médecins libéraux, maisons de santé) et utilisables par ces derniers c'est-à-dire s'adaptant à leurs contraintes en termes de mobilité du domicile ou de la chambre d'un patient à un(e) autre donc facilement transportable (c'est-à-dire respectant les contraintes de volume et de poids compatibles avec un transport « à la main »).

On atteint au moins l'un des objectifs précités avec un appareil portatif d'acquisition d'images d'une surface cutanée selon la revendication 1. Cet appareil comprend :

- une caméra d'acquisition d'images,
- un support mémoire pour stocker les images acquises par la caméra,
- plusieurs diodes électroluminescentes ou DELs pour éclairer une zone commune de la surface cutanée, ces diodes électroluminescentes comprenant des diodes électroluminescentes blanches et de couleur,
- une alimentation.

L'appareil selon l'invention comprend en outre un capteur de distance pour mesurer la distance entre la caméra et la surface cutanée, ainsi qu'une unité de traitement connectée au capteur de distance et configurée pour signaler lorsque la surface cutanée se trouve dans la profondeur de champ de la caméra.

Idéalement la profondeur de champ correspond à une plage de distances où l'image acquise est nette.

L'appareil selon l'invention permet l'acquisition d'images qui sont utilisées pour déterminer le niveau d'oxygénation cutanée.

Il s'agit d'imagerie sans contact, c'est-à-dire que l'appareil se trouve à plusieurs centimètres de la surface cutanée. Il ne touche pas la surface cutanée. Il est utilisable à main levée pour acquérir une séquence vidéo constituée d'images nettes.

Avec l'appareil selon l'invention, un capteur de distance permet de positionner la caméra à sa distance focale, plus globalement dans la profondeur de champ. On assure ainsi la netteté des images acquises.

La distance focale dépend de plusieurs paramètres : la taille du capteur de la caméra mais pour un capteur donné, le « milieu » ou « centre » de cette « plage de distances » est donnée par la distance focale propre à chaque lentille, c'est-à-dire l'objectif situé devant le capteur ; et pour une distance focale donnée, cette profondeur de champ dépend enfin de l'ouverture du diaphragme, par exemple f/11.

Avantageusement, un diaphragme est placé devant la caméra pour augmenter la profondeur de champ. Ce diaphragme présente de préférence une petite ouverture, par exemple inférieur à f/8, de préférence égale à f/11.

La caméra peut être de type monochrome mais elle est de préférence en couleur de façon à obtenir une image en couleurs réelles qui seules permettent un excellent indice de rendu colorimétrique.

Selon une caractéristique avantageuse de l'invention, le capteur de distance peut être un télémètre à ultrasons ou un télémètre à rayonnement infrarouge. Ce dernier est apte à envoyer un signal qui est ensuite réfléchi par la surface cutanée. La distance entre la caméra, sur laquelle est montée le télémètre, et la surface cutanée est déterminée à partir du temps passé entre l'émission et la réception du signal. Un tel télémètre est simple d'utilisation et peu coûteux. Selon une caractéristique avantageuse de l'invention, l'appareil peut comprendre au moins une diode ou un dispositif d'affichage pour générer un signal visible en réponse à une consigne provenant de l'unité de traitement lorsque la surface cutanée se trouve dans la profondeur de champ de la caméra.

Le signal visible peut être l'allumage ou l'extinction d'une diode, l'affichage d'un élément tel un motif, une image ou autre sur un dispositif d'affichage de la caméra ou un dispositif d'affichage rapporté.

Avantageusement, l'appareil selon l'invention peut comprendre un hautparleur apte à émettre un signal sonore en réponse à une consigne provenant de l'unité de traitement lorsque la surface cutanée se trouve dans la profondeur de champ de la caméra.

Que ce soit un signal sonore ou un signal visuel, l'appareil est capable d'indiquer à l'utilisateur que la distance qui sépare la caméra de la surface cutanée est convenable pour une prise d'image de qualité, nette, en utilisant notamment les diodes électroluminescentes de couleur.

La présence des diodes électroluminescentes de couleur et la capacité à mesurer la distance permet à l'appareil portable à une seule main d'acquérir à main levée (sans nécessité de recourir à l'usage d'un dispositif de type espaceur pour disposer le système à une distance fixe de la surface cutanée comme on le trouve sur certains dispositifs de visualisation utilisés en dermatologie) des séquences vidéo d'une durée de plusieurs secondes et constituées d'images nettes du début à la fin de la séquence vidéo.

Afin d'améliorer encore la qualité de l'image, l'appareil selon l'invention peut également comprendre un pare-soleil pour protéger l'objectif de la caméra de l'éclairage ambiant. Cela permet de préserver l'indice de rendu colorimétrique optimisé obtenu par l'éclairage blanc combinant une bonne uniformité d'éclairage sur toute la surface éclairée et une température de couleur corrélée optimisée.

Par l'indice de rendu de couleur, ou IRC, on entend l'évaluation quantitative du degré d'accord entre la couleur psychologique d'un objet éclairé par l'illuminant en essai et celle du même objet éclairé par l'illuminant de référence, l'état d'adaptation chromatique ayant été correctement pris en compte (Commission Internationale de l'Eclairage, Vocabulaire International de l'Eclairage, CIE S 017/E:2011, Vienne) .

L'IRC est quantitativement mesurable et s'exprime, pour une lumière considérée, par rapport à un idéal et pour une température de couleur donnée. L'indice maximum IRC=100 correspond ainsi à une lumière blanche « idéale » telle que la lumière naturelle, tandis que les indices faibles IRC<20 correspondent à la lumière émise par une lampe monochromatique, telle qu'une ampoule à vapeur de sodium, qui ne permet que peu de distinction des couleurs entre elles. Pour un éclairage de bonne qualité (comme c'est requis pour les expertises médicales), il est conseillé d'utiliser des lampes dont l'IRC est supérieur à 90.

Par ailleurs, pour obtenir des couleurs fiables sur toute la surface de mesure, il est avantageux d'avoir un éclairage uniforme. L'uniformité U peut être définie de la manière suivante :

$$U = \frac{I_{max} - I_{min}}{I_{max} + I_{min}}$$

$I_{max}$ (respectivement $I_{min}$) est défini comme étant l'intensité maximum (resp minimum) trouvée dans le champ de vision (selon les valeurs de niveaux de gris évalués en utilisant le logiciel Matlab 2014, The Mathworks, Inc.). Des valeurs plus petites de U produisent une meilleure uniformité d'éclairage. Des tests avec l'appareil selon l'invention ont permis d'obtenir des valeurs de U égales à 0,17 alors que des appareils selon l'art antérieur présentaient des valeurs de U jusqu'à 0,7, c'est en cela que l'appareil selon l'invention est dit optimisé.

Ainsi, le pare-soleil limite la luminosité provenant des alentours, pas uniquement de la lumière du soleil. Il contribue au fort indice de rendu colorimétrique caractérisant l'image qui permet de visualiser la morphologie de la surface cutanée.

Ce pare-soleil est un cache qui peut mesurer environ 3 cm de longueur autour de l'anneau d'éclairage et de la caméra afin de rendre l'image enregistrée indépendante de l'éclairage ambiant ; problème qui n'existe pas pour des dispositifs ayant la caméra posée au contact de la surface cutanée (tels que les dermoscopes par exemple). Une caractérisation de l'Indice de Rendu Colorimétrique (IRC) a permis de montrer l'efficacité de cette solution technologique comparativement à l'IRC de dispositifs posés au contact.

Selon une caractéristique avantageuse de l'invention, l'unité de traitement peut être configurée pour commander l'acquisition des images selon une séquence vidéo d'au moins une image par seconde. L'appareil selon l'invention permet de discriminer efficacement différents niveaux d'oxygénation cutanée.

Pour obtenir des images exploitables pour le calcul du niveau d'oxygénation, une durée optimale d'éclairage de la surface cutanée par chaque groupe de DELs (blanches ou de couleur) a été déterminée. Cela revient à déterminer la durée de "pause" de la caméra entre deux images pour l'accumulation d'un nombre de photons suffisant pour atteindre une sensibilité suffisante, le tout dans l'objectif de trouver le compromis idéal permettant une acquisition suffisamment rapide pour obtenir une cadence vidéo, au sens d'au moins 1 image par seconde.

Cette cadence d'une image par seconde permet un temps de pause suffisamment long pour accumuler assez de photons mais suffisamment court pour respecter une cadence suffisante pour être appelée "vidéo". Idéalement, on prévoit une cadence de dix images par secondes.

Avantageusement, l'unité de traitement peut être configurée pour commander l'acquisition des images en multiplexant temporellement l'éclairage entre les diodes électroluminescentes blanches et les diodes électroluminescentes de couleur. Ce multiplexage est obtenu de façon matérielle à partir d'un multiplexeur associé à l'unité de traitement ou bien de façon logicielle par traitement au sein de l'unité de traitement.

Selon une caractéristique avantageuse de l'invention, les diodes électroluminescentes de couleur comprennent des diodes électroluminescentes émettant dans la bande spectrale du rouge et des diodes électroluminescentes émettant dans la bande spectrale de l'infra-rouge.

La caméra d'acquisition d'images peut avantageusement être optimisée dans la bande spectrale proche infrarouge. Par optimisée, on entend un gain d'environ 20 points, typiquement entre 10 et 20, de pourcentage de sensibilité (efficacité quantique) pour des longueurs d'onde comprises entre 600 et 900 nm par rapport à une caméra "non-optimisée".

Les images obtenues à partir de la lumière blanche donnent accès à de l'information morphologique. On peut prévoir d'allumer une ou plusieurs diodes électroluminescentes blanches disposées du même côté pour visualiser les reliefs de la surface cutanée.

Les images obtenues à partir de la lumière rouge et de la lumière infra-rouge donnent accès à de l'information fonctionnelle permettant d'évaluer le niveau d'oxygénation cutanée.

L'appareil selon l'invention permet d'acquérir des images selon trois types d'éclairage : blanc, rouge et infra-rouge.

Le multiplexage temporel d'une séquence vidéo peut alors avoir lieu en lumière blanche (information morphologique) et en lumières rouge et infrarouge (information fonctionnelle).

Selon une autre caractéristique de l'invention, l'unité de traitement peut être configurée pour activer alternativement les diodes électroluminescentes émettant dans la bande spectrale du rouge et les diodes électroluminescentes émettant dans la bande spectrale de l'infra-rouge. Cela revient notamment à intercaler une image de lumière blanche (information morphologique) entre deux images apportant l'information fonctionnelle c'est-à-dire créées à partir de l'éclairage en lumière rouge et de l'éclairage en lumière infra-rouge.

Avantageusement, chaque groupe de diodes électroluminescentes blanches, rouges ou infrarouges peut être positionné selon un angle spécifique à chaque groupe par rapport à l'axe optique de façon à obtenir un éclairage homogène sur une surface cutanée commune. En d'autres termes, l'angle d'implantation des DELs est propre aux caractéristiques optiques de chaque type de DEL et permet une excellente homogénéité d'éclairage sur une même surface cutanée commune.

Selon l'invention, on arrange trois types de DELs différentes (différentes en longueurs d'onde, en irradiance et en ouverture numérique) en suffisamment grand nombre pour atteindre un niveau d'irradiance suffisant à la distance de travail (distance entre la caméra et la surface cutanée) mais inférieur aux seuils fixés par la norme NF EN 62471:2008 selon la directive européenne relative aux dispositifs électro-médicaux 93/42/CE.

La distance de travail peut par exemple être de 5 cm.

Le nombre de DELs pour chaque groupe étant contraint par le niveau d'irradiance de chaque groupe pour diminuer le temps de pause nécessaire de la caméra et parvenir à une cadence de type vidéo. Typiquement la tâche lumineuse peut être un carré de 2 cm de côté.

L'orientation des DELs est notamment fonction de l'ouverture numérique des DELs.

Selon une caractéristique avantageuse de l'invention, l'unité de traitement est configurée pour calculer en temps réel le niveau d'oxygénation cutanée et l'afficher sous la forme d'un code couleur. Chaque couleur peut correspondre à une plage de valeurs de niveau d'oxygénation.

Contrairement à des systèmes de l'art antérieur qui apportent des informations du type concentrations en Hb, HbO2, etc., l'appareil selon l'invention fournit à l'utilisateur une information, la PtcO2, validée par les consensus cliniques internationaux comme faisant partie des paramètres à évaluer dans le cadre d'une procédure de prise en charge des plaies chroniques.

Selon l'invention, l'unité de traitement est configurée pour respecter un temps de pause après chaque acquisition d'image, ce temps de pause étant fonction de la sensibilité souhaitée dans la détermination du niveau d'oxygénation cutanée. Plus le temps de pause est élevé, plus la sensibilité est grande. Une sensibilité grande du niveau d'oxygénation est le fait de pouvoir déterminer de faibles valeurs de la pression partielle transcutanée d'oxygène, PtcO2.

Avec l'appareil selon l'invention, on peut réaliser une évaluation du niveau d'oxygénation cutanée d'une surface cutanée entière. A titre d'exemple, l'appareil peut réaliser une mesure de la PtcO2 sur une surface cutanée de 4 cm$^2$ par seconde de séquence vidéo, soit environ 40 cm$^2$ par 10 secondes, et non plus ponctuellement 3 cm$^2$ par 30 minutes comme en pratique clinique actuellement. De plus, le multiplexage temporel des 2 flux vidéo en lumière blanche et en lumière rouge/infra-

rouge permet d'envoyer dans le même temps avec le même appareil une image classique (porteuse de l'information morphologique) et une image de mesure de l'information du niveau d'oxygénation cutanée pour la même surface cutanée.

Selon une caractéristique avantageuse de l'invention, l'appareil peut comprendre un filtre polariseur circulaire polarisant verticalement la lumière provenant de la surface cutanée, ce filtre polariseur circulaire étant disposé entre l'objectif de la caméra et la surface cutanée pour supprimer une lumière due à la réflexion spéculaire provenant de la surface cutanée.

Selon une caractéristique avantageuse de l'invention, l'appareil peut comprendre un filtre polariseur linéaire polarisant horizontalement la lumière générée par une partie des diodes électroluminescentes blanches, ce filtre polariseur linéaire étant disposé uniquement face à ladite partie de diodes électroluminescentes blanches.

De préférence, les diodes électroluminescentes blanches sont disposées le long d'un cercle de diamètre D1, les diodes électroluminescentes de couleur étant disposées le long d'un cercle de diamètre D2 tel que D2>D1. Les deux cercles peuvent être concentriques.

Avantageusement, les diodes électroluminescentes peuvent être maintenues par une couronne disposée autour d'un objectif de la caméra. La couronne est un élément solide, rigide qui porte les DELs. Idéalement il s'agit donc d'une couronne circulaire, mais il peut être de forme carrée, rectangulaire, ovale ou autre plus complexe. Le but étant d'éclairer uniformément la surface cutanée.

Par ailleurs, le pare-soleil peut se fixer à la couronne et peut comporter une rainure sur sa paroi interne pour maintenir le film polarisant linéaire.

Selon une caractéristique avantageuse de l'invention, l'appareil peut comprendre une lentille de correction de linéarité pour limiter des distorsions dues à l'utilisation de différentes longueurs d'ondes parmi les diodes électroluminescentes.

Une lentille de correction de linéarité permet d'assurer la netteté de l'image selon les trois modes d'éclairage. Sachant que l'angle de diffraction dépend de la longueur d'onde, les angles de diffraction des rayonnements émis par les DELs blanches, rouges ou infrarouges sont très différents les uns par rapport aux autres.

L'appareil selon l'invention comprend un écran d'affichage et l'unité de traitement est configurée pour afficher simultanément un flux vidéo acquis en lumière blanche pour visualiser la morphologie de la surface cutanée et une représentation graphique du niveau d'oxygénation en niveaux de couleurs sur la surface cutanée avec affichage du taux moyen d'oxygénation. Cet écran peut donc être intégré à l'appareil tenant dans la main ou bien il peut s'agir d'un écran distant (écran d'ordinateur, téléviseur, téléphone portable, écran distant dédié, etc.) auquel le reste de l'appareil se connecte avec ou sans fil.

De préférence, l'appareil selon l'invention comprend un module de communication sans fil pour un transfert des images.

En complément notamment de ce qui précède, l'appareil selon l'invention peut comprendre un corps principal ayant à une extrémité une tête optique dans laquelle se trouvent au moins les diodes électroluminescentes, et un manche fixé à un côté latéral du corps principal. A titre d'exemple, l'alimentation peut être disposée dans le manche, alors que l'unité de traitement et la caméra peuvent être disposées dans le corps principal.

Suivant un autre aspect de l'invention, il est proposé un procédé selon la revendication 20.

Bien entendu, les différentes caractéristiques, formes et variantes de réalisation de l'invention peuvent être associées les unes avec les autres selon diverses combinaisons dans la mesure où elles ne sont pas incompatibles ou exclusives les unes des autres.

D'autres avantages et caractéristiques de l'invention apparaîtront à l'examen de la description détaillée d'un mode de mise en œuvre nullement limitatif, et des dessins annexés, sur lesquels :

La figure 1 est une vue schématique d'un appareil portatif sans fil pour l'acquisition de vidéos cutanées selon l'invention,

La figure 2 est une vue schématique de la tête optique de l'appareil selon l'invention,

La figure 3 est une vue schématique du filtre et du pare-soleil venant coiffer les diodes électroluminescentes de la tête optique selon l'invention,

La figure 4 est une vue en coupe schématique très simplifiée de la tête otique,

La figure 5 est une vue schématique très simplifiée en blocs fonctionnels de l'appareil selon l'invention,

La figure 6 est un organigramme illustrant un algorithme de calcul du niveau d'oxygénation selon l'invention,

La figure 7 est une image d'une surface cutanée sur une partie d'une main en utilisant la lumière blanche selon l'invention,

La figure 8 est une image traitée d'une surface cutanée sur une partie d'une main en utilisant une illumination rouge et infrarouge selon l'invention,

La figure 9 est une autre image traitée d'une surface cutanée sur deux doigts (l'un des deux doigts subissant une occlusion veineuse par la mise en place d'un garrot au niveau de l'articulation carpo-métacarpienne pour induire une hypoxie cutanée) en utilisant une illumination rouge et infrarouge selon l'invention, et

La figure 10 est une vue schématique de la tête de l'appareil selon un exemple de réalisation.

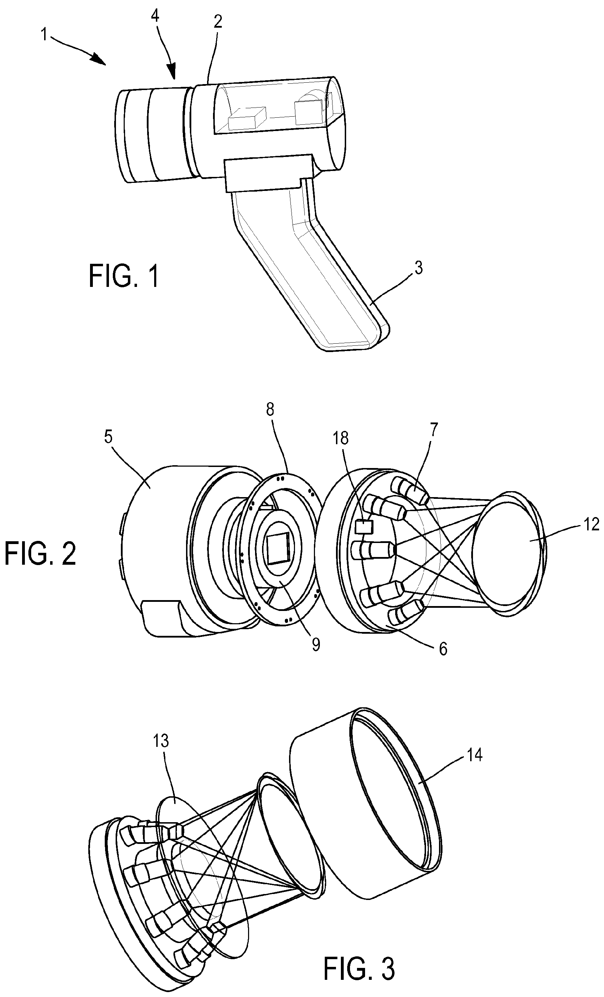

Sur la figure 1 on distingue un appareil 1 selon l'invention destiné à acquérir des vidéos d'une surface cutanée. Cet appareil 1 comprend un corps principal désigné dans sa globalité par la référence 2 et un manche 3.

Le corps principal 2 comprend une tête optique 4 dotée de moyens optiques nécessaires pour l'acquisition d'images. Une carte électronique est disposée dans le reste du corps principal. Ce dernier présente une forme cylindrique à section circulaire. Il s'agit donc d'un cylindre allongé d'une dizaine de centimètres et un rayon d'environ quatre centimètres. La tête optique est disposée sur l'extrémité avant, c'est la partie qui vient en face de la surface cutanée à imager lors d'une utilisation.

Le manche 3 est de dimension tenant dans la main. Il est fixé au corps principal sur un côté latéral. Lorsqu'un utilisateur tient le manche, le corps principal se trouve sur la partie supérieure. La forme globale de l'appareil est celle d'un pistolet. Le manche comporte un coude entre une partie supérieure et une partie inférieure. La partie supérieure est directement connectée au corps principal de façon perpendiculaire à la paroi extérieur du corps principal. La partie inférieure part ensuite en biais pour faciliter la préhension.

L'appareil selon l'invention est un dispositif portatif qui pèse moins de 1 kg et qui est transportable dans une petite valise du domicile d'un patient à un autre, comme c'est le cas pour un infirmier à domicile, ou de la chambre d'un résidant d'EHPAD à une autre.

En référence aux figures 2 à 4, on distingue quelques éléments de la tête optique 4.

La caméra 5 est utilisée pour sa sensibilité optimisée dans la bande spectrale visible - proche infra-rouge (470 - 850 nm). La caméra comprend un capteur 9 de type CMOS permettant d'enregistrer une séquence vidéo sur une carte mémoire insérée dans la caméra.

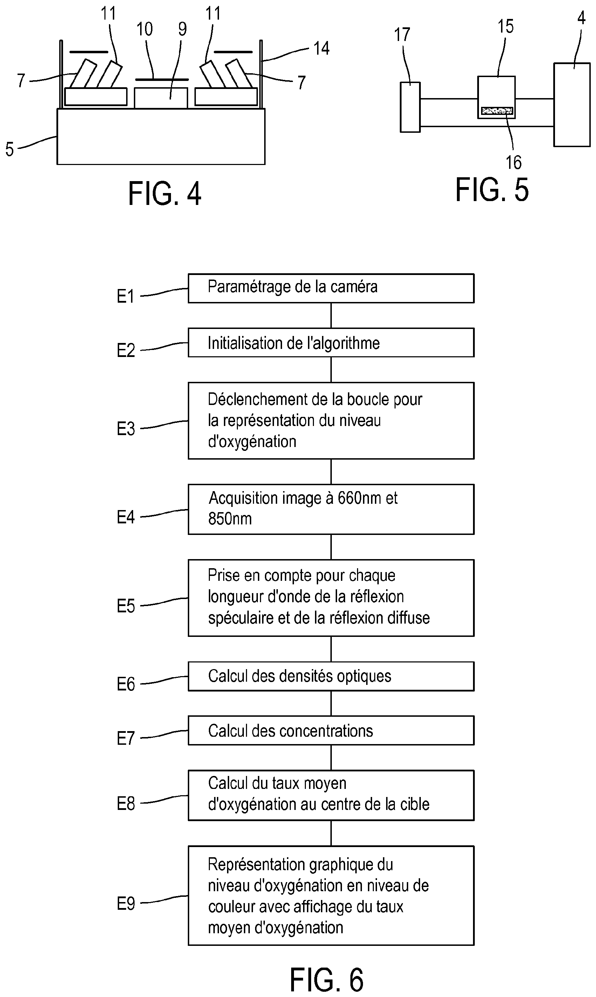

Ce capteur 9 est recouvert d'un filtre polariseur circulaire 10, visible sur la figure 4, sous forme de disque placé devant le capteur 9. Ce filtre polariseur circulaire 10 est d'un bleu transparent polarisant verticalement la lumière en provenance de la surface cutanée à imager. Il repose sur un bord spécifique faisant partie intégrante de la couronne 6.

Une couronne 6 est disposée sur la caméra 5, du côté extérieur et autour du capteur 9. La couronne porte huit DELs blanches 7 entourant le capteur CMOS. Pour des raisons de simplification du dessin, les DELs de couleurs ne sont pas représentées sur les figures 2 et 3, mais uniquement sur la figure 4 sous la référence 11. Chaque diode électroluminescente est insérée en force dans un trou orienté à 24° dans la couronne 6.

Un circuit d'alimentation 8 de forme circulaire est inséré dans la couronne 6 par l'arrière, du côté de la caméra 5. Il relie chaque DEL à l'alimentation et est contrôlé par l'unité de traitement pour allumer séquentiellement les DELs.

Les diodes électroluminescentes sont orientées de façon à éclairer de façon homogène la surface cutanée. Le disque 12 sur la figure 2 illustre de façon fictive la surface cutanée où les faisceaux de lumière des diodes électroluminescentes se superposent en une surface d'éclairage homogène.

Un filtre polariseur linéaire 13 est prévu, dans lequel des trous ont été percés de manière à disposer :

- d'un éclairage polarisé lorsque quatre DELs blanches sont allumées, il s'agit de DELs devant lesquelles le filtre est effectivement présent, non troué,
- d'un éclairage non-polarisé lorsque les quatre autres DELs blanches sont allumées, celles faisant face aux trous percés dans le filtre.

Le filtre polariseur linéaire polarise horizontalement la lumière générée par les diodes électroluminescentes blanches.

On prévoit également un pare-soleil 14 qui se fixe sur la couronne et comporte un rebord pour maintenir le filtre polariseur linéaire en place.

Sur la figure 5, la tête optique est globalement référencée en 4, qui en association avec le reste de l'appareil, permet d'obtenir une séquence vidéo dont les images présentent un indice de rendu colorimétrique élevé.

Une unité de traitement 15 permet de contrôler l'allumage des diodes électroluminescentes selon une cadence prédéterminée. Par exemple en ce qui concerne les DELs blanches, l'unité de traitement peut allumer successivement quatre DELs pour disposer de l'éclairage polarisé puis quatre autres DELs pour disposer de l'éclairage non-polarisé, et enfin deux DELs situées du même côté afin de disposer d'un éclairage latéral visant à offrir une meilleure visualisation des reliefs cutanés. Les deux dernières DELs sont prises parmi l'ensemble des DELs blanches prévues pour réaliser des images polarisées ou non, mais il peut également s'agir de deux DELs blanches supplémentaires.

Selon l'invention, l'appareil comprend un capteur de distance 18 pour mesurer la distance entre la caméra et la surface cutanée. Ce capteur de distance est un télémètre à ultrasons qui permet de positionner la caméra à sa distance focale, plus ou moins la profondeur de champ de façon à assurer la netteté de l'image.

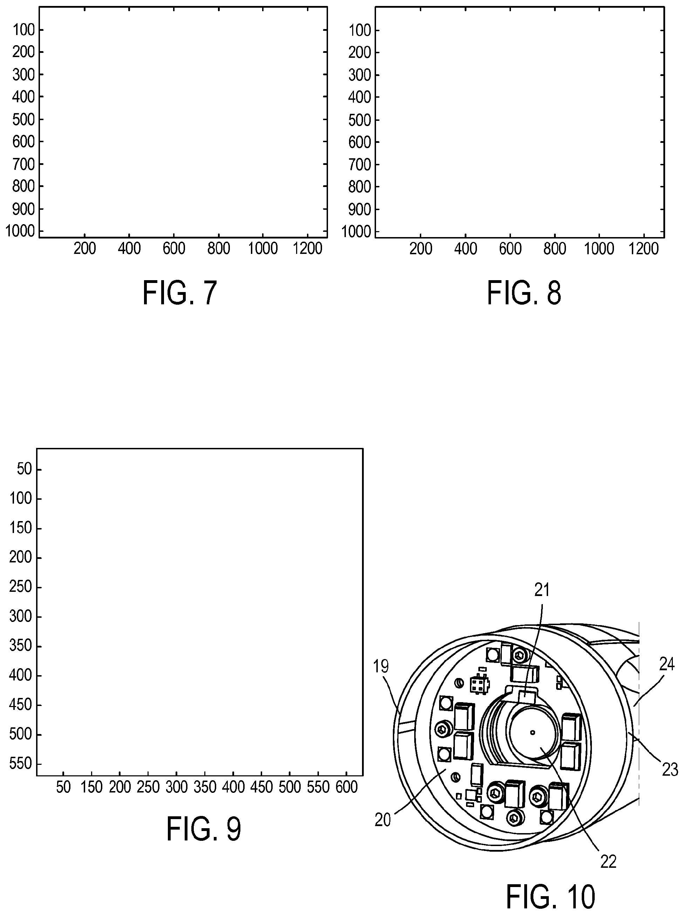

Avantageusement, ce capteur de distance est connecté à l'unité de traitement qui allume en orange un indicateur lumineux (voir figure 10) disposé sur l'appareil si le télémètre détecte une distance trop petite entre caméra et la surface cutanée, en bleu si la distance est trop grande et en vert si la distance est comprise entre deux extrémum qui correspondent à la profondeur de champ maximale et minimale.

Ce capteur de distance 18 peut être disposé sur la couronne comme on le voit sur la figure 2, mais il peut également être disposé sur la caméra, intégré à la caméra ou bien disposé sur une paroi externe du corps principal. De préférence, le télémètre est disposé le plus près possible de la caméra.

De préférence, la focale de la caméra est fixe avec une profondeur de champ (par exemple avec un diaphragme en f/11) permettant de modifier en temps réel la distance de travail dans la limite de la profondeur de champ. On peut également envisager une focale variable - autofocus.

L'unité de traitement peut comprendre un ou plusieurs microprocesseurs ou microcontrôleurs associés à des composants matériels et/ou logiciels pour le bon fonctionnement de l'appareil selon l'invention. La partie logicielle est apte à interagir avec le microprocesseur, la caméra, le circuit d'alimentation, à traiter les images acquises et à afficher deux flux vidéos.

Sur la figure 5 on distingue également un module de communication sans fil 16 pour la communication avec un appareil distant tel un serveur, un routeur, un téléphone portable, un ordinateur, une tablette ou autre. Ce module de communication sans fil permet avantageusement de transférer des images et vidéos depuis l'appareil vers l'extérieur.

Sur la figure 5 on distingue également une alimentation 17.

Selon l'invention l'alimentation peut être en deux parties. Une batterie qui alimente l'unité de traitement, et une pile rechargeable qui alimente les DELs.

La batterie peut être disposée dans le manche 3 de l'appareil. La pile rechargeable peut être disposée dans le corps principal 2.

L'ensemble de l'appareil tient en un seul volume car tous les éléments sont assemblés et protégés par des coques en plastique.

Sur la figure 6 est illustré un algorithme codé pour piloter l'allumage séquentiel des réseaux de DELs, synchroniser l'acquisition vidéo, réaliser les calculs nécessaires à l'affichage multiplexé dans le temps des deux flux vidéo d'intérêt.

L'étape E1 de paramétrage de la caméra comprend les opérations suivantes :

- Ouverture de la caméra,
- Configuration du mode d'affichage bitmap
- Configuration des couleurs en 8 bit
- Réglage de la gamme et de l'offset
- Réglage du temps d'exposition, du nombre d'images par seconde
- Configuration du déclenchement de la caméra
- Allocation de la mémoire pour les images
- Test d'acquisition des images par la caméra

L'étape E2 d'initialisation de l'algorithme permet de définir des coefficients d'extinction de Hb (hémoglobine), $HbO_2$ (oxy-hémoglobine) pour les longueurs d'onde de travail, 660 nm et 850 nm. Cela permet également de définir des profondeurs de pénétration de la lumière dans la peau, ainsi que de définir des taux de réflexion spéculaire et diffuse. Puis initialisation au cours de la même étape, de cinq matrices de même taille que celles des images à acquérir :

- 2 matrices de densités optiques à 660 nm et 850 nm,
- 2 matrices pour la concentration en Hb et celle en HbO$_2$,
- Une matrice du niveau d'oxygénation de la peau.

Ensuite c'est l'étape E3 qui permet de déclencher la boucle pour la représentation du niveau d'oxygénation.

L'étape E4 concerne l'acquisition d'images à 660 nm et à 850 nm.

A l'étape E5, il s'agit de la prise en compte pour chaque longueur d'onde de la réflexion spéculaire et de la réflexion diffuse.

Le calcul des densités optiques (OD) est réalisé à l'étape E6.

Le calcul des concentrations est réalisé à l'étape E7 selon notamment la formule suivante :

$$C_{HbO_2} = \frac{OD_{\lambda_2} - \frac{\varepsilon_{Hb_{\lambda_2}}.d_{\lambda_2}}{\varepsilon_{Hb_{\lambda_1}}.d_{\lambda 1}}.OD_{\lambda_1}}{\varepsilon_{HbO2_{\lambda_2}}.d_{\lambda_2} - \varepsilon_{Hb_{\lambda_2}}.d_{\lambda_2}.\frac{\varepsilon_{HbO2_{\lambda_1}}}{\varepsilon_{Hb_{\lambda_1}}}}$$

$$C_{Hb} = \frac{OD_1}{\varepsilon_{Hb_{\lambda_1}}.d_{\lambda_1}} - \frac{\varepsilon_{HbO2_{\lambda_2}}}{\varepsilon_{Hb_{\lambda_2}}}.C_{HbO_2}$$

Le calcul du taux moyen d'oxygénation au centre de la cible est réalisé à l'étape E8.

Puis, à l'étape E9, est réalisée une représentation graphique du niveau d'oxygénation en niveaux de couleurs avec affichage du taux moyen d'oxygénation.

Les figures 7 et 8 illustrent deux images qui présentent respectivement deux flux vidéo affichés simultanément produits par l'appareil selon l'invention.

La figure 7 montrant le flux vidéo acquis en lumière blanche et qui donne accès à l'information morphologique. La figure 8 montre le flux vidéo acquis en alternant illumination rouge (660 nm) et illumination infrarouge (850 nm) qui après traitement des données permet d'afficher une image donnant les niveaux d'oxygénation cutanée aussi appelés information fonctionnelle.

La figure 9 montre la capacité de l'appareil selon l'invention à détecter une variation de niveau d'oxygénation cutanée en comparant le niveau d'oxygénation cutanée de deux doigts d'une même main : le majeur présentant un niveau d'oxygénation « normal » (à gauche sur la figure avec une couleur unie dominante) et l'index auquel est appliqué une occlusion veineuse au niveau de l'articulation carpo-métacarpienne présentant en conséquence de l'occlusion un niveau d'oxygénation cutanée inférieur (à droite sur la figure avec une importante partie centrale nuancée).

Selon un aspect particulièrement avantageux de l'invention, l'appareil :

- est portatif et sans fil, lui permettant d'être utilisable plus facilement dans certaines situations de télémédecine,
- permet d'acquérir à main levée des images contenant des informations utiles car les images sont d'une grande netteté grâce notamment au télémètre permettant de positionner correctement la caméra dans la profondeur de champ du capteur,
- permet d'acquérir une image classique caractérisée par un fort indice de rendu colorimétrique grâce au fait que le système intègre :

  - un éclairage lumineux dont la température de couleur corrélée est optimisée (par exemple pour discriminer des couples de couleurs d'intérêts en dermatologie clinique), et
  - un pare-soleil qui isole efficacement le capteur de l'éclairage environnant.

- permet d'acquérir simultanément deux types d'information colocalisés (morphologique et fonctionnelle) qui peuvent être transmises à distance,
- est compatible avec une commercialisation car est d'un coût de fabrication maîtrisé,
- est compatible avec une utilisation en clinique car respecte les exigences essentielles de la directive européenne 93/42/CE relative aux dispositifs électro-médicaux notamment en termes de compatibilité électromagnétique et de sécurité électrique et photobiologique, et
- permet de disposer de plusieurs modes de visualisation :

  - Le mode polarisé, c'est-à-dire qui supprime la brillance souvent gênante pour la visualisation des lésions cutanées sur une image photo ou vidéo (la brillance étant le nom commun employé pour désigner le phénomène optique de la réflexion spéculaire),
  - Le mode classique sans polarisation dans les cas où celui-ci présenterait un intérêt,
  - La visualisation latérale afin de rendre plus évidents les reliefs.

La figure 10 est une vue schématique de la tête de l'appareil selon un autre exemple de réalisation de l'appareil selon l'invention. On distingue un pare-soleil 19 jouant le même rôle que le pare-soleil 14 des figures 3 et 4. Une carte électronique 20 porte un ensemble de composants électroniques tels que par exemple des condensateurs, des résistances et des circuits de commande.

On distingue le télémètre 21 selon l'invention disposé à proximité de la caméra 22 permettant de détecter la distance entre la caméra et la surface cutanée. Des diodes de signalisation sont disposées sur le pourtour de la carte électronique 20 de façon à éclairer une bande circulaire 23, visible de l'extérieur de l'appareil et disposée entre le pare-soleil 19 et le reste 24 de la tête de l'appareil.

On peut également envisager de disposer dans la tête de l'appareil de pointeurs laser (de classe 1 par exemple).

Des pointeurs laser pourraient dessiner les coins d'un carré de visualisation afin que l'utilisateur visualise facilement la surface cutanée dont il est en train d'acquérir l'image sans avoir besoin de déplacer son regard vers un écran d'ordinateur sur lequel la vidéo est projetée simultanément. Il s'agit d'un moyen ergonomique pour que l'utilisateur sache exactement et en temps réel la surface cutanée dont il a acquis l'image.

D'une façon générale, l'invention concerne un appareil portatif d'acquisition d'images d'une surface cutanée, cet appareil comprenant :

- une caméra d'acquisition d'images, vidéo ou images fixes,
- un support mémoire pour stocker les images acquises par la caméra,
- plusieurs diodes électroluminescentes pour éclairer de manière multiplexée dans le temps une zone commune de la surface cutanée avec une homogénéité optimisée. Ces diodes électroluminescentes comprennent des diodes électroluminescentes blanches à température de couleur corrélée optimisée destinées à l'acquisition d'images en couleurs réelles avec fort indice de rendu colorimétrique et des diodes électroluminescentes de couleur destinées à la production d'images renseignant sur le niveau d'oxygénation cutanée.

Par température de couleur corrélée (TCC) optimisée, on entend une TCC (mesurée par exemple par un spectroradiomètre et exprimée en Kelvin) incluse dans la plage de valeurs solaires, c'est-à-dire comprise entre 5 000 et 6 000 K.

Par fort indice, on entend un IRC supérieur à 90, par exemple de 94,4 sachant que le maximal théorique est 100.

Les niveaux d'irradiance peuvent notamment respecter tous les seuils fixés par la norme NF EN 62471:2008 selon la directive européenne relative aux dispositifs électro-médicaux 93/42/CE.

Selon l'invention, les deux types d'images sont affichées simultanément à l'écran.

L'appareil comprend également :

- une alimentation, et
- un capteur de distance pour mesurer la distance entre la caméra et la surface cutanée autorisant une manipulation à main levée et permettant ainsi l'acquisition d'une séquence vidéo constituée d'images toutes nettes.

L'appareil peut en outre comprendre un pare-soleil (contribuant lui aussi à l'indice de rendu colorimétrique élevé) une unité de traitement connectée au capteur de distance et configurée pour signaler lorsque la surface cutanée se trouve dans la profondeur de champ de la caméra.

Bien sûr, l'invention n'est pas limitée aux exemples qui viennent d'être décrits et de nombreux aménagements peuvent être apportés à ces exemples sans sortir du cadre de l'invention.

**Revendications**

1. Appareil (1) portatif d'acquisition d'images d'une surface cutanée, cet appareil comprenant :

    - une caméra (5) d'acquisition d'images,
    - un support mémoire pour stocker les images acquises par la caméra,
    - plusieurs diodes électroluminescentes (7, 11) pour éclairer une zone (12) commune de la surface cutanée, ces diodes électroluminescentes comprenant des diodes électroluminescentes blanches et de couleur,
    - une alimentation (8),
    - un capteur de distance (18) pour mesurer la distance entre la caméra (5) et la surface cutanée (12),
    - une unité de traitement (15) connectée au capteur de distance (18) et configurée pour signaler lorsque la surface cutanée se trouve dans la profondeur de champ de la caméra (5),

    **caractérisé en ce qu'**il comprend un écran d'affichage et l'unité de traitement (15) est configurée pour afficher simultanément un flux vidéo acquis en lumière blanche pour visualiser la morphologie de la surface cutanée (12) et une représentation graphique du niveau d'oxygénation en niveaux de couleurs sur la surface cutanée avec affichage du taux moyen d'oxygénation.

2. Appareil selon l'une quelconque des revendications précédentes, **caractérisé en ce que** les diodes électroluminescentes de couleur (17) comprennent des diodes électroluminescentes émettant dans la bande spectrale du rouge et des diodes électroluminescentes émettant dans la bande spectrale de l'infra-rouge.

3. Appareil selon la revendication 2, **caractérisé en ce que** l'unité de traitement (15) est configurée pour activer alternativement les diodes électroluminescentes émettant dans la bande spectrale du rouge et les diodes électroluminescentes émettant dans la bande spectrale de l'infra-rouge.

4. Appareil selon la revendication 2 ou 3, **caractérisé en ce que** chaque groupe de diodes électroluminesc-

centes blanches, rouges ou infrarouges est positionné selon un angle spécifique à chaque groupe par rapport à l'axe optique de façon à obtenir un éclairage homogène sur une surface cutanée commune.

5. Appareil selon l'une quelconque des revendications précédentes, **caractérisé en ce qu'**un diaphragme est placé devant la caméra (5) pour augmenter la profondeur de champ.

6. Appareil selon l'une quelconque des revendications précédentes, **caractérisé en ce qu'**il comporte une lentille de correction de linéarité pour limiter des distorsions dues à l'utilisation de différentes longueurs d'ondes parmi les diodes électroluminescentes.

7. Appareil selon l'une quelconque des revendications précédentes, **caractérisé en ce que** le capteur de distance (18) est un télémètre à ultrasons ou à rayonnement infrarouge.

8. Appareil selon l'une quelconque des revendications précédentes, **caractérisé en ce qu'**il comprend au moins une diode ou un dispositif d'affichage pour générer un signal visible en réponse à une consigne provenant de l'unité de traitement (15) lorsque la surface cutanée se trouve dans la profondeur de champ de la caméra.

9. Appareil selon l'une quelconque des revendications précédentes, **caractérisé en ce qu'**il comprend un hautparleur apte à émettre un signal sonore en réponse à une consigne provenant de l'unité de traitement lorsque la surface cutanée se trouve dans la profondeur de champ de la caméra.

10. Appareil selon l'une quelconque des revendications précédentes, **caractérisé en ce qu'**il comprend un pare-soleil (14, 19) pour protéger l'objectif de la caméra de l'éclairage ambiant.

11. Appareil selon l'une quelconque des revendications précédentes, **caractérisé en ce que** l'unité de traitement (15) est configurée pour commander l'acquisition des images selon une séquence vidéo d'au moins une image par seconde.

12. Appareil selon l'une quelconque des revendications précédentes, **caractérisé en ce que** l'unité de traitement (15) est configurée pour commander l'acquisition des images en multiplexant temporellement l'éclairage entre les diodes électroluminescentes blanches et les diodes électroluminescentes de couleur.

13. Appareil selon l'une quelconque des revendications précédentes, **caractérisé en ce que** l'unité de traitement (15) est configurée pour calculer en temps réel le niveau d'oxygénation cutanée et l'afficher selon un code couleur.

14. Appareil selon la revendication 13, **caractérisé en ce que** l'unité de traitement (15) est configurée pour respecter un temps de pause après chaque acquisition d'image, ce temps de pause étant proportionnel à une précision souhaitée sur la détermination du niveau d'oxygénation cutanée.

15. Appareil selon l'une quelconque des revendications précédentes, **caractérisé en ce que** les diodes électroluminescentes blanches sont disposées le long d'un cercle de diamètre D1, les diodes électroluminescentes de couleur étant disposées le long d'un cercle de diamètre D2 tel que D2>D1.

16. Appareil selon la revendication 10, **caractérisé en ce que** le pare-soleil (14,19) se fixe à une couronne (6) et comporte une rainure sur sa paroi interne pour maintenir le film polarisant linéaire (13).

17. Appareil selon l'une quelconque des revendications précédentes, **caractérisé en ce qu'**il comprend un module de communication sans fil (16) pour un transfert des images.

18. Appareil selon l'une quelconque des revendications précédentes, **caractérisé en ce qu'**il comprend un corps principal (2) ayant à une extrémité une tête optique (4) dans laquelle se trouvent au moins les diodes électroluminescentes, et un manche (3) fixé à un côté latéral du corps principal.

19. Appareil selon la revendication 18, **caractérisé en ce que** l'alimentation est disposée dans le manche, (3) alors que l'unité de traitement (15) et la caméra (5) sont disposées dans le corps principal (2).

20. Procédé d'acquisition d'images d'une surface cutanée à partir d'un appareil (1), dans lequel procédé on réalise les étapes suivantes :

> - acquisition d'images à partir d'une caméra d'acquisition (5),
> - stockage des images acquises sur un support mémoire,
> - éclairage d'une zone commune (12) de la surface cutanée à partir de plusieurs diodes électroluminescentes (7, 11), ces diodes électroluminescentes comprenant des diodes électroluminescentes blanches et de couleur,
> - mesure de la distance entre la caméra et la surface cutanée au moyen d'un capteur de distance, (18) et
> - émission d'un signal en utilisant une unité de traitement (15) lorsque la surface cutanée se trouve dans la profondeur de champ de la caméra,

- **caractérisé en ce qu'**il comprend une étape d'affichage simultanément d'un flux vidéo acquis en lumière blanche pour visualiser la morphologie de la surface cutanée (12) et d'une représentation graphique du niveau d'oxygénation en niveaux de couleurs sur la surface cutanée avec affichage du taux moyen d'oxygénation.

**Patentansprüche**

1. Tragbares Gerät (1) zur Erfassung von Bildern einer Hautoberfläche, wobei dieses Gerät umfasst:

   - eine Kamera (5) zur Erfassung von Bildern,
   - ein Speichermedium zum Abspeichern der von der Kamera erfassten Bilder,
   - mehrere Leuchtdioden (7, 11) zum Beleuchten eines gemeinsamen Bereichs (12) der Hautoberfläche, wobei die Leuchtdioden weiße und farbige Leuchtdioden umfassen,
   - eine Stromversorgung (8),
   - einen Entfernungssensor (18) zum Messen der Entfernung zwischen der Kamera (5) und der Hautoberfläche (12),
   - eine Verarbeitungseinheit (15), die mit dem Entfernungssensor (18) verbunden und dazu ausgelegt ist, zu melden, wenn die Hautoberfläche innerhalb der Schärfentiefe der Kamera (5) liegt,

   **dadurch gekennzeichnet, dass** es einen Anzeigebildschirm umfasst und die Verarbeitungseinheit (15) dazu ausgelegt ist, gleichzeitig einen mit Weißlicht erfassten Videostream zur Visualisierung der Morphologie der Hautoberfläche (12) und eine grafische Darstellung des Sauerstoffversorgungsgrads in Farbwerten auf der Hautoberfläche mit Anzeige des durchschnittlichen Sauerstoffversorgungsgrads anzuzeigen.

2. Gerät nach einem der vorhergehenden Ansprüche, **dadurch gekennzeichnet, dass** die farbigen Leuchtdioden (17) Leuchtdioden umfassen, die im roten Spektralband emittieren, und Leuchtdioden, die im Infrarot-Spektralband emittieren.

3. Gerät nach Anspruch 2, **dadurch gekennzeichnet, dass** die Verarbeitungseinheit (15) dazu ausgelegt ist, abwechselnd die im roten Spektralband emittierenden Leuchtdioden und die im Infrarot-Spektralband emittierenden Leuchtdioden einzuschalten.

4. Gerät nach Anspruch 2 oder 3, **dadurch gekenn-**

**zeichnet, dass** jede Gruppe von weißen, roten oder Infrarot-Leuchtdioden in einem für jede Gruppe spezifischen Winkel zur optischen Achse positioniert ist, um eine homogene Beleuchtung auf einer gemeinsamen Hautoberfläche zu erzielen.

5. Gerät nach einem der vorhergehenden Ansprüche, **dadurch gekennzeichnet, dass** vor der Kamera (5) eine Blende angeordnet ist, um die Schärfentiefe zu erhöhen.

6. Gerät nach einem der vorhergehenden Ansprüche, **dadurch gekennzeichnet, dass** es eine Linse zur Linearitätskorrektur enthält, um Verzerrungen aufgrund der Verwendung unterschiedlicher Wellenlängen unter den Leuchtdioden zu begrenzen.

7. Gerät nach einem der vorhergehenden Ansprüche, **dadurch gekennzeichnet, dass** der Entfernungssensor (18) ein Ultraschall- oder Infrarotstrahlungs-Entfernungsmesser ist.

8. Gerät nach einem der vorhergehenden Ansprüche, **dadurch gekennzeichnet, dass** es mindestens eine Diode oder eine Anzeigevorrichtung umfasst, um ein sichtbares Signal als Reaktion auf eine Anweisung von der Verarbeitungseinheit (15) zu erzeugen, wenn die Hautoberfläche innerhalb der Schärfentiefe der Kamera liegt.

9. Gerät nach einem der vorhergehenden Ansprüche, **dadurch gekennzeichnet, dass** es einen Lautsprecher umfasst, der in der Lage ist, ein Tonsignal als Reaktion auf eine Anweisung von der Verarbeitungseinheit zu erzeugen, wenn die Hautoberfläche innerhalb der Schärfentiefe der Kamera liegt.

10. Gerät nach einem der vorhergehenden Ansprüche, **dadurch gekennzeichnet, dass** es eine Sonnenblende (14, 19) umfasst, um das Kameraobjektiv von der Umgebungsbeleuchtung abzuschirmen.

11. Gerät nach einem der vorhergehenden Ansprüche, **dadurch gekennzeichnet, dass** die Verarbeitungseinheit (15) dazu ausgelegt ist, die Erfassung der Bilder in einer Videosequenz von mindestens einem Bild pro Sekunde zu steuern.

12. Gerät nach einem der vorhergehenden Ansprüche, **dadurch gekennzeichnet, dass** die Verarbeitungseinheit (15) dazu ausgelegt ist, die Erfassung der Bilder durch zeitliches Multiplexen der Beleuchtung zwischen den weißen Leuchtdioden und den farbigen Leuchtdioden zu steuern.

13. Gerät nach einem der vorhergehenden Ansprüche, **dadurch gekennzeichnet, dass** die Verarbeitungseinheit (15) dazu ausgelegt ist, in Echtzeit den Sauerstoffversorgungsgrad der Haut zu berechnen und farbcodiert anzuzeigen.

14. Gerät nach Anspruch 13, **dadurch gekennzeichnet, dass** die Verarbeitungseinheit (15) dazu ausgelegt ist, nach jeder Bilderfassung eine Pausenzeit einzuhalten, wobei diese Pausenzeit proportional zu einer gewünschten Genauigkeit bei der Bestimmung des Sauerstoffversorgungsgrads der Haut ist.

15. Gerät nach einem der vorhergehenden Ansprüche, **dadurch gekennzeichnet, dass** die weißen Leuchtdioden entlang eines Kreises mit dem Durchmesser D1 angeordnet sind, wobei die farbigen Leuchtdioden entlang eines Kreises mit dem Durchmesser D2 angeordnet sind, wobei D2 > D1 gilt.

16. Gerät nach Anspruch 10, **dadurch gekennzeichnet, dass** die Sonnenblende (14, 19) an einem Kranz (6) befestigt ist und an ihrer Innenwand eine Nut aufweist, um den linearen Polarisationsfilm (13) zu halten.

17. Gerät nach einem der vorhergehenden Ansprüche, **dadurch gekennzeichnet, dass** es ein drahtloses Kommunikationsmodul (16) für eine Bildübertragung umfasst.

18. Gerät nach einem der vorhergehenden Ansprüche, **dadurch gekennzeichnet, dass** es einen Hauptkörper (2), der an einem Ende einen optischen Kopf (4) aufweist, in dem sich zumindest die Leuchtdioden befinden, und einen Griff (3) umfasst, der an einer lateralen Seite des Hauptkörpers befestigt ist.

19. Gerät nach Anspruch 18, **dadurch gekennzeichnet, dass** die Stromversorgung im Griff (3) angeordnet ist, während die Verarbeitungseinheit (15) und die Kamera (5) im Hauptkörper (2) angeordnet sind.

20. Verfahren zur Erfassung von Bildern einer Hautoberfläche mit einem Gerät (1), wobei in diesem Verfahren die folgenden Schritte ausgeführt werden:

   - Erfassen von Bildern mit einer Erfassungskamera (5),
   - Abspeichern der erfassten Bilder auf einem Speichermedium,
   - Beleuchten eines gemeinsamen Bereichs (12) der Hautoberfläche mit mehreren Leuchtdioden (7, 11), wobei diese Leuchtdioden weiße und farbige Leuchtdioden umfassen,
   - Messen der Entfernung zwischen der Kamera und der Hautoberfläche mithilfe eines Entfernungssensors (18), und
   - Ausgeben eines Signals unter Verwendung einer Verarbeitungseinheit (15), wenn die Hautoberfläche innerhalb der Schärfentiefe der Kamera liegt,
   - **dadurch gekennzeichnet, dass** es einen Schritt des gleichzeitigen Anzeigens von einem mit Weißlicht erfassten Videostream zur Visualisierung der Morphologie der Hautoberfläche (12) und einer grafischen Darstellung des Sauerstoffversorgungsgrads in Farbwerten auf der Hautoberfläche mit Anzeige des durchschnittlichen Sauerversorgungsgrads umfasst.

## Claims

1. A portable apparatus (1) for acquiring images of a skin surface, this apparatus comprising:

   - an image acquisition camera (5),
   - a storage medium for storing images acquired by the camera,
   - multiple light-emitting diodes (7, 11) for illuminating a common area (12) of the skin surface, these light-emitting diodes comprising white and colored light-emitting diodes,
   - a power supply (8),
   - a distance sensor (18) for measuring the distance between the camera (5) and the skin surface (12),
   - a processing unit (15) connected to the distance sensor (18) and configured to signal when the skin surface is within the depth of field of the camera (5),
   **characterized in that** it comprises a display screen and the processing unit (15) is configured to simultaneously display a video stream acquired in white light to visualize the morphology of the skin surface (12) and a graphic representation of the oxygenation level in color levels on the skin surface with a display of the average oxygenation rate.

2. The apparatus according to any one of the preceding claims, **characterized in that** the colored light-emitting diodes (17) comprise light-emitting diodes emitting in the red spectral band and light-emitting diodes emitting in the infrared spectral band.

3. The apparatus according to claim 2 **characterized in that** the processing unit (15) is configured to alternately activate the light-emitting diodes emitting in the red spectral band and the light-emitting diodes emitting in the infrared spectral band.

4. The apparatus according to claim 2 or 3, **characterized in that** each group of white, red or infrared light-emitting diodes is positioned at an angle specific to each group with respect to the optical axis so as to obtain homogeneous illumination on a common skin surface.

**5.** The apparatus according to any one of the preceding claims, **characterized in that** a diaphragm is placed in front of the camera (5) to increase the field depth.

**6.** The apparatus according to any one of the preceding claims, **characterized in that** it comprises a linearity correction lens to limit distortions due to the use of different wavelengths from the light-emitting diodes.

**7.** The apparatus according to any one of the preceding claims, **characterized in that** the distance sensor (18) is an ultrasonic or infrared rangefinder.

**8.** The apparatus according to any one of the preceding claims, **characterized in that** it comprises at least one diode or a display device for generating a visible signal in response to a command from the processing unit (15) when the skin surface is within the depth of field of the camera.

**9.** The apparatus according to any one of the preceding claims, **characterized in that** it comprises a loudspeaker capable of emitting a sound signal in response to a command from the processing unit when the skin surface is within the depth of field of the camera.

**10.** The apparatus according to any one of the preceding claims, **characterized in that** it comprises a lens hood (14, 19) to protect the camera lens from ambient lighting.

**11.** The apparatus according to any one of the preceding claims, **characterized in that** the processing unit (15) is configured to control the acquisition of images according to a video sequence of at least one image per second.

**12.** The apparatus according to any one of the preceding claims, **characterized in that** the processing unit (15) is configured to control the acquisition of images by time-multiplexed illumination between the white light-emitting diodes and the colored light-emitting diodes.

**13.** The apparatus according to any one of the preceding claims, **characterized in that** the processing unit (15) is configured to calculate the skin oxygenation level in real time and display it according to a color code.

**14.** The apparatus according to claim 13, **characterized in that** the processing unit (15) is configured to respect a pause time after each image acquisition, this pause time being proportional to a desired precision on the determination of the skin oxygenation level.

**15.** The apparatus according to any one of the preceding claims, **characterized in that** the white light-emitting diodes are arranged along a circle of diameter D1, the colored light-emitting diodes being arranged along a circle of diameter D2 such that D2>D1.

**16.** The apparatus according to claim 10, **characterized in that** the lens hood (14, 19) is attached to a ring (6) and comprises a groove on the inner wall thereof to hold the linear polarizing film (13).

**17.** The apparatus according to any one of the preceding claims, **characterized in that** it comprises a wireless communication module (16) for image transfer.

**18.** The apparatus according to any one of the preceding claims, **characterized in that** it comprises a main body (2) having at one end an optical head (4) wherein at least the light-emitting diodes are located, and a handle (3) attached to a lateral side of the main body.

**19.** The apparatus according to claim 18, **characterized in that** the power supply is arranged in the handle (3), while the processing unit (15) and the camera (5) are arranged in the main body (2).

**20.** A method of acquiring images of a skin surface from an apparatus (1), in which method the following steps are performed:

- acquiring images from an acquisition camera (5),
- storing acquired images on a storage medium,
- illuminating a common area (12) of the skin surface from multiple light-emitting diodes (7, 11), these light-emitting diodes comprising white and colored light-emitting diodes,
- measuring the distance between the camera and the skin surface by means of a distance sensor (18), and
- emitting a signal using a processing unit (15) when the skin surface is within the depth of field of the camera,
- **characterized in that** it comprises a step of simultaneously displaying a video stream acquired in white light to visualize the morphology of the skin surface (12) and a graphical representation of the oxygenation level in color levels on the skin surface with a display of the average oxygenation rate.

FIG. 1

FIG. 2

FIG. 3

FIG. 4

FIG. 5

| | |
|---|---|
| E1 | Paramétrage de la caméra |
| E2 | Initialisation de l'algorithme |
| E3 | Déclenchement de la boucle pour la représentation du niveau d'oxygénation |
| E4 | Acquisition image à 660nm et 850nm |
| E5 | Prise en compte pour chaque longueur d'onde de la réflexion spéculaire et de la réflexion diffuse |
| E6 | Calcul des densités optiques |
| E7 | Calcul des concentrations |
| E8 | Calcul du taux moyen d'oxygénation au centre de la cible |
| E9 | Représentation graphique du niveau d'oxygénation en niveau de couleur avec affichage du taux moyen d'oxygénation |

FIG. 6

**FIG. 7**

**FIG. 8**

**FIG. 9**

**FIG. 10**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2004174525 A
- US 20130071103 A
- US 2014240468 A
- US 2016022181 A
- US 2015124067 A
- US 2011117025 A

**Littérature non-brevet citée dans la description**

- **KONG, L. et al.** Non-contact detection of oxygen saturation based on visible light imaging device using ambient light. *Opt. Express*, 2013, vol. 21, 17464-17471
- **NITZAN, M. et al.** Calibration-Free Pulse Oximetry Based on Two Wavelengths in the Infrared - A Preliminary Study. *Sensors*, 2014, vol. 14, 7420-7434